# EUROPÄISCHE PATENTSCHRIFT

(11) **EP 0 299 429 B1**
(45) Veröffentlichungstag und Bekanntmachung des Hinweises auf die Patenterteilung: **19.01.1994**
(21) Anmeldenummer: 88111137.1
(22) Anmeldetag: 12.07.1988
(51) Int. Cl.: A61K 35/44, A61K 35/48

(54) **Verfahren zur Herstellung eines Präparates auf Grundlage von Immunabwehrzellen aus der Wharton-Sulze**
Preparation of a composition comprising immune cells from Wharton's jelly
Procédé de préparation d'une composition à base de cellules de défense immunitaire extraites de la gelée de Wharton

(30) Priorität: 13.07.1987 DE 3723132
(43) Veröffentlichungstag der Anmeldung: 18.01.1989
(73) Patentinhaber: Schusteritz, Ludwig, Dr., D-63755 Alzenau (DE)
(72) Erfinder: Schusteritz, Ludwig, Dr., D-63755 Alzenau (DE)
(74) Vertreter: Barz, Peter, Dr.

(56) Entgegenhaltungen:
- EP-A- 0 012 774
- DE-A- 3 326 387
- FR-A- 2 413 912
- FR-M- 1 957
- Römpps Chemie-Lexikon, 8. Auflage, Band 2, S. 1229-1230
- Lehrbuch der pharmazeutischen Technologie, R. Voigt, 5. Auflage, S. 450

## Beschreibung

Das Immunsystem ist ein Schutzmechanismus, der dem menschlichen Körper Integrität gegen Bakterien- und Virusinfektionen, sowie gegen körperfremde Schadsubstanzen gewährleistet. Gegen die zivilisationsbedingte Immunschwäche, die eine zunehmende Anfälligkeit des Menschen für Viruserkrankungen und Krebs zur Folge hat, sind bekannte Heilverfahren nur sehr unbefriedigend wirksam. Die bei schweren derartigen Erkrankungen kausal allein wirksame Therapie durch Substitution von biologisch aktiven Immunabwehrzellen ist noch nicht realisierbar, da es derzeit nur gelingt, Abwehrzellfraktionen, beispielsweise Lymphozyten, aus menschlichem Blut zu isolieren. Die Komplexität der Immunabwehrvorgänge erfordert jedoch für eine effektive Behandlung derartiger Erkrankungen eine Vielfalt unterschiedlicher Abwehrzellen. Die Anwendung von Abwehrzellfraktionen liefert daher nur unbefriedigende Therapie-Ergebnisse und ist darüber hinaus sehr aufwendig und teuer.

Es ist bekannt, Abwehrzellfraktionen in Zellkulturen zu züchten. Diese als monoklonale Antikörper bezeichneten Zellen besitzen jedoch nur eine sehr eng begrenzte Erkennungsfähigkeit für Viren und Krebszellen, so daß die Identifizierungsrate für Viren und Krebszellen aufgrund deren Mutationsfreudigkeit nur gering und die Heilergebnisse entsprechend mäßig sind.

Es ist ferner aus der DE-PS 12 37 730 bekannt, daß man ein Krebsheilmittel erhält, wenn man Bindegewebszellen in Form von Frischzellen in isotoner Lösung mit Heparin und Dextran umsetzt.

Es wurde nun gefunden, daß in dem als Wharton-Sulze bezeichneten Teil der Säugetier-Nabelschnur biologisch hochaktive Immunabwehrzellen in großer Zahl und Vielfalt vorhanden sind. Sie besitzen ein hohes Erkennungsvermögen für Antigene jeglicher Art, insbesondere für Viren und Krebszellen. Diese Abwehrzellen sind in einer fetalen, amorphen Grundsubstanz eingebettet, die von maternalen, immunologisch hochdifferenzierten Bindegewebszellen umgeben und durchsetzt ist.

Werden solche Immunabwehrzellen aus der Wharton-Sulze in den menschlichen Körper injiziert, so besteht die Gefahr einer Immunreaktion bis hin zum anaphylaktischen Schock.

In FR-M-1957 werden Organextrakte aus verschiedenen Organen (nicht aber der Nabelschnur oder spezieller der Wharton-Sulze) beschrieben. In FR-A-2 413 912 wird ein pharmazeutisches Präparat beschrieben, das unter anderem aus einem löslichen Extrakt aus der Wharton-Sulze bestehen kann. EP-A-0 012 774 offenbart lösliche Extrakte aus der menschlichen Nabelschnur. In DE-A-3326387 wird ein Frischzellenextrakt aus der gesamten Nabelschnur etwa 4 Monate alter Bergschaf-Feten offenbart, dessen Gewinnung nur pauschal beschrieben wird, nämlich daß die Nabelschnur unter sterilen Bedingungen in Ringerlösung gereinigt, zerkleinert und durch ein Sieb passiert wird und die so gewonnenen Frischzellen mit Pannet-Compton-Lösung vermischt werden.

Aufgabe der vorliegenden Erfindung ist es daher, ein verbessertes Präparat auf der Grundlage von Immunabwehrzellen aus der Wharton-Sulze zu schaffen, welches immunologisch indifferent ist.

Gegenstand der Erfindung ist ein Verfahren zur Herstellung eines Präparats auf Grundlage von Immunabwehrzellen aus der Wharton-Sulze von Säugetierfeten, bei dem man Säugetierfeten zu einem Zeitpunkt, in dem die Feten noch immunologisch indifferent sind, die Nabelschnur entnimmt, den Teil der Nabelschnur, der die Wharton-Sulze enthält, abtrennt und daraus eine isotone wäßrige Zellsuspension herstellt, indem man die Wharton-Sulze in eingefrorenem Zustand zerkleinert, die erhaltenen Gewebsstücke in einer bei 0°C gekühlten, isotonen Salzlösung verrührt, sie in dieser Lösung mit einem hochtourigen Gerät mit rotierenden Messern weiter zerkleinert, die nicht schwebfähigen Zellgewebsanteile abtrennt und die resultierende Zellsuspension gewinnt.

Die in dem erfindungsgemäßen Verfahren verwendeten Immunabwehrzellen aus der Wharton-Sulze der Nabelschnur von Säugetieren können z. B. dadurch erhalten werden, daß man die immunologisch indifferenten, fetalen Abwehrzellen von dem Allergien auslösenden maternalen Zellmaterial abtrennt. Hierzu entnimmt man die Nabelschnur einem Säugetierfetus, vorzugsweise einem Rinder- oder Schaffetus, zu einem Zeitpunkt, in dem der Fetus noch immunologisch indifferent ist. Die immunologische Indifferenz kann z.B. dadurch bestimmt werden, daß man dem Fetus entnommene Zellen geeigneten Versuchstieren verabfolgt und prüft, ob eine entsprechende Immunreaktion auftritt oder unterbleibt. Vorzugsweise werden die Immunabwehrzellen Feten entnommen, die ein Alter von höchstens 3/4, vorzugsweise von 1/5 bis 3/4 der Gestationszeit haben. Die Abtrennung des die Wharton-Sulze enthaltenden Teils von der Nabelschnur eines Säugetierfetus erfolgt vorzugsweise unter sterilen Bedingungen. Dieser die Wharton-Sulze enthaltende Teil der Nabelschnur wird mit einem geeigneten Gerät, z.B. einem Messer oder einer Schere, vorzerkleinert. Zu diesem Zweck ist es erfindungsgemäß notwendig, die Wharton-Sulze zuvor einzufrieren, vorzugsweise mittels flüssigem Stickstoff zu schockgefrieren. Die erhaltenen Gewebsstücke werden in einer auf ca. 0°C vorgekühlten, isotonen Salzlösung verrührt. Als isotone Salzlösung eignet sich z.B. eine sogenannte Ringerlösung.

Anschließend wird eine weitere Zerkleinerung durchgeführt. Zu diesem Zweck bedient man sich einer geeigneten Schneidevorrichtung, vorzugsweise einem hochtourigen Gerät mit rotierenden Messern, z.B. einem Ultra-Turrax-Gerät. Nach dieser Feinzerkleinerung kann die entstandene Zellsuspension von nicht schwebfähigen Anteilen durch geeignete Maßnahmen, im allgemeinen nicht-chemische Maßnahmen, befreit werden. Dies gelingt z.B. mittels eines Siebes von geeigneter Porengröße mit oder ohne Zuhilfenahme von Verbandsgaze, einer Filtration oder Zentrifugation. Die so entstandene, mäßig trübe Zellsuspension kann nach einigem Stehen vom Bodensatz abgetrennt werden.

Das erhaltene Immunabwehrzellkonzentrat wird dann in geeigneter Weise weiterverarbeitet, z.B. indem man es einem Gefriertrocknungsprozeß unterzieht oder in sterile Einmalspritzen aufzieht und mittels eines geeigneten Kühlmittels, vorzugsweise flüssigem Stickstoff, in der Weise schockgefriert, daß das Wasser im Zytoplasma der Abwehrzellen amorph erstarrt.

Die erfindungsgemäß hergestellten Abwehrzell-Präparate sind biologisch hoch wirksam, ohne Aktivitätsverlust lange haltbar, und können als Diagnostica, Prophylaktica und Therapeutica verwendet werden.

Die erfindungsgemäßen Präparate sind nach parenteraler Verabreichung bei durch Bakterien oder Viren, wie beispielsweise Grippe-, Herpes- oder Aids-Viren, verursachten Infektionserkrankungen wirksam. Sie eignen sich weiterhin für die Substitutionstherapie bei Immunabwehrinsuffizienz sowie zur Erkennung, Verhütung und Behandlung maligner Erkrankungen und besonders zum direkten Abbau maligner Tumore unterschiedlicher Art.

Weiterhin ist es überraschend, daß die Immunabwehrzellen aus der Wharton-Sulze eine für ein körperfremdes Präparat aus tierischen Zellen ungewöhnlich hohe Verträglichkeit, insbesondere auch bei der Injektionsbehandlung am Menschen, zeigen. Die erfindungsgemäßen Präparate sind im Abstand von wenigen Tagen ohne Allergiegefahr auch mehrfach applizierbar. Eine immunsupressive Vorbehandlung, wie sie bei der Übertragung körperfremden Zellmaterials derzeit noch unerlässlich ist, ist bei den erfindungsgemäßen Präparaten nicht erforderlich.

Die therapeutische Wirksamkeit der erfindungsgemäßen, Immunabwehrzellen aus der Wharton-Sulze von Tierfeten enthaltenden Präparate kann noch dadurch gesteigert werden, daß ihnen Thymusdrüsen-Extrakte zugemischt werden. Dies geschicht vorzugsweise durch Zugabe von Thymusdrüsen-Extrakten zu dem erfindungsgemäßen Präparat vor der Injektion. Die Thymuswirkstoffe können so im engsten Kontakt mit den thymuskompetenten Rezeptoren der Abwehrzellen reagieren, wodurch deren Erkennungsfähigkeit für Viren und Krebszellen erheblich gesteigert bzw. überhaupt erst ermöglicht wird. Damit gelingt es, maligne Tumore, vor allem aber auch deren Metastasen, durch die von den Abwehrzellen bewirkte Phagozytose abzubauen, ohne daß zusätzliche radiologische oder chemotherapeutische Behandlungen nötig sind. Die Heilwirkung erstreckt sich sowohl auf zytotoxische als auch viral induzierte Krebserkrankungen, während gutartige Tumore nur sehr langsam oder gar nicht beeinflußt werden. Da der Angriff der Abwehrzell-Thymusextrakt-Kombinationsprodukte sehr schnell erfolgt und sich am Patienten an den durch Krebs erkrankten Stellen des Körpers durch charakteristische Sensationen bemerkbar macht, ist durch diese Präparate auch eine einfache und preiswerte Krebs-Diagnose möglich, die nicht nur die Existenz maligner Prozesse, sondern auch deren Lokalisation und Progredienz anzuzeigen vermag.

Die folgenden Beispiele dienen zur Eräuterung der vorliegenden Erfindung.

### Beispiel 1

60 g frische oder auf -196°C tiefgefrorene, die Wharton-Sulze enthaltende Nabelschnur werden mittels Messer oder Schere in dünne Scheiben zerteilt. Die erhaltenen Gewebsstücke werden in 200 ml Ringerlösung von 0°C suspendiert und mit Hilfe eines Ultra-Turrax-Gerätes stark zerkleinert. Nach Zugabe von weiteren 300 ml gekühlter Ringerlösung wird die erhaltene Suspension durch ein Sieb gegossen und die ablaufende, feinere Suspension erneut mittels eines Siebes mit mehrfacher Auflage von Verbandsmull von den gröberen Gewebsanteilen befreit. Das feintrübe Filtrat wird anschließend ca. 10 Minuten bei 0°C aufbewahrt und anschließend vom gebildeten Bodensatz durch Dekantieren abgetrennt. Daraufhin wird die erhaltene Zellsuspension in sterile Einmalspritzen aufgezogen. Der Spritzeninhalt wird mittels flüssigem Stickstoff auf -196°C abgekühlt und bei Temperaturen unter -80°C gelagert. Alle Arbeitsgänge erfolgen unter sterilen Bedingungen.

### Beispiel 2

Die nach Beispiel 1 nach Dekantieren vom Bodensatz erhaltene feintrübe Zellsuspension wird zentrifugiert. Die als Sediment nach Abtrennung der überstehenden Flüssigkeit verbleibenden Abwehrzellen werden in üblicher Weise gefriergetrocknet.

## Patentansprüche

1. Verfahren zur Herstellung eines Präparats auf Grundlage von Immunabwehrzellen aus der Wharton-Sulze von Säugetierfeten, bei dem man Säugetierfeten zu einem Zeitpunkt, in dem die Feten noch immunologisch indifferent sind, die Nabelschnur entnimmt, den Teil der Nabelschnur, der die Wharton-Sulze enthält, abtrennt und daraus eine isotone wäßrige Zellsuspension herstellt, indem man die Wharton-Sulze in eingefrorenem Zustand zerkleinert, die erhaltenen Gewebsstücke in einer bei 0°C gekühlten, isotonen Salzlösung verrührt, sie in dieser Lösung mit einem hochtourigen Gerät mit rotierenden Messern weiter zerkleinert, die nicht schwebfähigen Zellgewebsanteile abtrennt und die resultierende Zellsuspension gewinnt.

2. Verfahren nach Anspruch 1, bei dem Feten mit einem Alter von höchstens 3/4 der Gestationszeit verwendet werden.

3. Verfahren nach Anspruch 1 oder 2, bei dem die Wharton-Sulze von Rinder- oder Schafsfeten verwendet wird.

4. Verfahren nach einem der Ansprüche 1 bis 3, bei dem die resultierende Zellsuspension lyophilisiert oder schockgefroren wird.

5. Verfahren nach einem der Ansprüche 1 bis 4, bei dem man der Zellsuspension zusätzlich Thymusdrüsen-Extrakt zumischt.

## Claims

1. A process for the production of a preparation on the basis of immune defence cells from Wharton's jelly of mammal fetuses, which comprises taking the umbilical cord from mammal fetuses at a point of time at which the fetuses are still immunologically indifferent, removing that part of the umbilical cord which contains the Wharton jelly and processing it to obtain an aqueous isotonic cell suspension by comminuting the Wharton jelly in a frozen state, stirring the tissue fragments obtained in an isotonic salt solution cooled at 0°C, further comminuting them in said solution by means of a high-speed device having rotating knifes, removing the non-floatable cell tissue components and recovering the resultant cell suspension.

2. The process of claim 1, wherein fetuses having an age of at most 3/4 of the gestation period are used.

3. The process of claim 1 or 2, wherein Wharton's jelly from bovine or ovine fetuses is used.

4. The process of any one of claims 1 to 3, wherein the cell suspension obtained is lyophilized or shock-frozen.

5. The process of any one of claims 1 to 4, wherein the cell suspension is additionally admixed with thymus gland extract.

## Revendications

1. Procédé de préparation d'une composition à base de cellules de défense immunitaire extraites de la gelée de Wharton de foetuses mammifères, comprenant le prélèvement du cordon ombilical des foetuses mammifères à une date à laquelle les foetuses sont encore immunologiquement indifférents, la séparation du part du cordon ombilical qui contient la gelée de Wharton et la préparation d'une suspension de cellules aqueuse et isotonique par broyage de la gelée de Wharton en état congelé, agitation des pièces de tissu obtenues dans une solution saline isotonique et refroidie à 0°C, broyage supplémentaire des pièces dans ladite solution avec un appareil ayant des couteaux tournants a grande vitesse, séparation des composantes de tissu cellulaire non-flottantes et obtention de la suspension de cellules résultante.

2. Procédé suivant la revendication 1, dans lequel des foetuses d'un âge correspondent au maximum à 3/4 de la période de gestation sont utilisés.

3. Procédé suivant la revendication 1 ou 2, dans lequel la gelée de Wharton de foetuses bovines ou ovines est utilisée.

4. Procédé suivant l'une des revendications 1 à 3, dans lequel la suspension de cellules obtenue est lyophilisée ou choc-congelée.

5. Procédé suivant l'une des revendications 1 à 4, dans lequel d'extrait de thymus est additionné à la suspension de cellules.
